# EUROPEAN PATENT SPECIFICATION

(11) **EP 1 857 055 B1**
(45) Date of publication and mention of the grant of the patent: **06.04.2016**
(21) Application number: 07252081.0
(22) Date of filing: 21.05.2007
(51) Int. Cl.: A61B 17/04

(54) **Suture locking device**
Wundnahtverschlussvorrichtung
Dispositif de blocage de sutures

(30) Priority: 19.05.2006 US 437440
(43) Date of publication of application: 21.11.2007
(73) Proprietor: ETHICON ENDO-SURGERY, INC., Cincinnati, Ohio 45242 (US)
(72) Inventor: Stokes, Michael J., Cincinnati Ohio 45244 (US); Conlon, Sean P., Cincinnati Ohio 45140 (US); Holcomb, Matthew D., Loveland Ohio 45140 (US)
(74) Representative: Tunstall, Christopher Stephen

(56) References cited:
- WO-A-00/12014
- WO-A-99/04699
- US-A- 6 086 608
- US-A1- 2002 188 321
- US-A1- 2003 167 062
- US-A1- 2005 216 040

## Description

### FIELD OF THE INVENTION

The present description relates in general to endoscopic surgical devices and, more particularly, to a suture locking device for severing and securing the ends of a suture material within a body cavity during an endoscopic surgical procedure.

The invention is set out in the appended claims that follow.

### BACKGROUND OF THE INVENTION

Endoscopic procedures have been rapidly developing over the past decade. These procedures often allow for the performance of surgical procedures with minimal trauma when compared to prior techniques requiring a large external opening to expose the internal organ or tissue requiring repair.

In addition to the many areas in which endoscopic procedures have found use, endoscopic procedures have been developed for surgical procedures addressing morbid obesity. Morbid obesity is a serious medical condition. In fact, morbid obesity has become highly pervasive in the United States, as well as other countries, and the trend appears to be heading in a negative direction. Complications associated with morbid obesity include hypertension, diabetes, coronary artery disease, stroke, congestive heart failure, multiple orthopedic problems and pulmonary insufficiency with markedly decreased life expectancy. With this in mind, and as those skilled in the art will certainly appreciate, the monetary and physical costs associated with morbid obesity are substantial. In fact, it is estimated the costs relating to obesity are in excess of 100 billion dollars in the United States alone.

A variety of surgical procedures have been developed to treat obesity. One procedure is Roux-en-Y gastric bypass (RYGB). This operation is highly complex and is commonly utilized to treat people exhibiting morbid obesity. Around 35,000 procedures are performed annually in the United States alone. Other forms of bariatric surgery include Fobi pouch, bilio-pancreatic diversion, and gastroplasty or "stomach stapling". In addition, implantable devices are known which limit the passage of food through the stomach and affect satiety.

RYGB involves movement of the jejunum to a high position using a Roux-en-Y loop. The stomach is completely divided into two unequal portions (a smaller upper portion and a larger lower gastric pouch) using an automatic stapling device. The upper pouch typically measures less than about 1 ounce (or 20 cc), while the larger lower pouch remains generally intact and continues to secret stomach juices flowing through the intestinal track.

A segment of the small intestine is then brought from the lower abdomen and joined with the upper pouch to form an anastomosis created through a half-inch opening, also called the stoma. This segment of the small intestine is called the "Roux loop" Roux limb and carries the food from the upper pouch to the remainder of the intestines, where the food is digested. The remaining lower pouch and the attached segment of duodenum are then reconnected to form another anastomotic connection to the Roux loop limb at a location approximately 50 to 150 cm from the stoma, typically using a stapling instrument. It is at this connection that the digestive juices from the bypass stomach, pancreas, and liver, enter the jejunum and ileum to aide in the digestion of food. Due to the small size of the upper pouch, patients are forced to eat at a slower rate and are satiated much more quickly. This results in a reduction in caloric intake.

As those skilled in the art will certainly appreciate, the conventional RYGB procedure requires a great deal of operative time. Because of the degree of invasiveness, post-operative recovery time can be quite lengthy and painful. In view of the highly invasive nature relating to the current RYGB procedure, other less invasive procedures have been developed. With this mind other procedures for reducing the size of the stomach have been developed. The most common form of gastric reduction surgery involves the application of vertical staples along the stomach to create an appropriate pouch. This procedure is commonly performed laparoscopically and as such requires substantial preoperative, operative, postoperative resources.

As endoscopic devices and procedures have developed, surgeons have begun to employ endoscopic techniques to gastric procedures such as those discussed above in an effort to minimize trauma and reduce the time required for procedures and recovery. With the foregoing in mind, procedures and apparatuses that allow for the performance of gastric reduction surgery in a time efficient and patient friendly manner are needed.

One area that has not been adequately addressed is the need for the application of sutures as these gastric, and other endoscopic, procedures are being performed. The present description provides an endoscopic suturing device adapted for the continuous application of sutures. US 2002/188321; US 2005/216040; US 6 086 608: WO99/04699; WO 00/12014 and US 2003/167062 describe different suture locking devices comprising an adapter and a knot lying element.

### SUMMARY OF THE INVENTION

In accordance with the present description there is provided a suture locking device including an adapter for insertion into a body. The adapter has a proximal end, an open distal end and a lip within the distal end projecting towards an interior of the open distal end. The device further includes a knot tying element releasably attached to the distal end of the adapter. The knot tying element has a distal and a proximal end. The proximal end of the knot tying element has a recess thereon. The proximal end of the knot tying element is inserted within the open distal end of the adapter such that the lip and the recess mate with each other to form a releasable lock.

The invention is set out in the appended claims. The examples, aspects or embodiments of the present description that do not fall within the scope of said claims are provided for illustrative purposes only and do not form part of the present invention.

### BRIEF DESCRIPTION OF THE DRAWINGS

The present invention will be better understood by reference to the following description, taken in conjunction with the accompanying drawings, in which:
FIG. 1 is an isometric view of a first embodiment for a suture locking device in an unfired position;
FIG. 2 is a longitudinal, cross-sectional view of the suture locking device of FIG. 1;
FIG. 3 is an exploded, isometric view of the suture locking device of FIG. 1;
FIG. 4A is an isolated, isometric view of the inner locking member of FIG.3;
FIG. 4B is a cross-sectional view of the inner locking member of FIG. 4A, taken along line B-B;
FIG. 5A is an isolated, isometric view of the outer locking member shown in FIG. 3;
FIG. 5B is a cross-sectional view of the outer locking member shown in FIG. 5A, taken along line B-B;
FIG. 6 is an isometric view of the adaptor and the anchor section of the launching member prior to attachment;
FIG. 7A is a cross-sectional view of the suture locking device in an initial loading stage;
FIG. 7B is a cross-sectional view of the suture locking device, in which the launching member is being inserted into the adaptor during loading;
FIG. 7C is a cross-sectional view of the suture locking device at the end stage of loading, wherein the launching member has been lowered into axial alignment with the adaptor and housing;
FIG. 8A is an isometric view of a launching member modified in accordance with an alternative embodiment for attaching the launching member to a drive cable;
FIG. 8B is an isometric view showing the launching member of FIG. 8A attached to the distal end of a ferrule and modified adaptor;
FIG. 9 is a plan view of an exemplary deployment handle for the suture locking device;
FIG. 10 is a cross-sectional view of the deployment handle of FIG. 9;
FIG. 11 is a cross-sectional view of the suture locking device of FIG. 1, showing the device threaded with suture material prior to firing;
FIG. 12 is a cross-sectional view similar to FIG. 11, showing the inner and outer locking members engaging during the initial stages of firing;
FIG. 13 is a cross-sectional view of the device of FIG. 11, showing the components of the knotting element engaged just prior to fracture; and
FIG. 14 is a cross-sectional view similar to FIG. 11, showing the suture material severed and the knotting element detached from the device post firing.

### DETAILED DESCRIPTION OF THE INVENTION

During an endoscopic surgical procedure an endoscope, containing onboard visualization, is passed through a body orifice to reach a surgical site. Using the onboard visualization, small-diameter flexible instruments are manipulated at the site to join tissue segments together. Typically, the tissue segments are joined with a thin, flexible suturing material such as thread, wire or the like. Following the joining procedure, the suture material is secured in place to prevent the tissue from separating. During an open surgical procedure, in which a larger incision is made to accommodate instruments, suture materials are oftentimes joined by tying knots at the loose ends of the material. Various devices have been developed to assist the surgeon in tying knots during surgical procedures, including suture clip-type devices. In endoscopic procedures, however, knot tying can be difficult and time-consuming due to the small available working area within the endoscope. Oftentimes, the resulting knots lack the adequate holding strength or tightness to maintain the tissue junction. Accordingly, it is desirable to provide a suture locking device that can effectively be used in the confined space available in an endoscopic procedure. Additionally, it is desirable to provide a suture locking device that provides for in-line tensioning of the suture material prior to joining the suture ends together. Further, it is desirable to provide a suture locking device that can be reloaded and reused to position multiple suture knotting elements during a procedure.

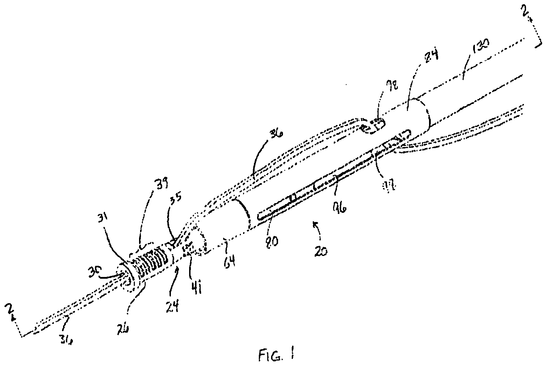

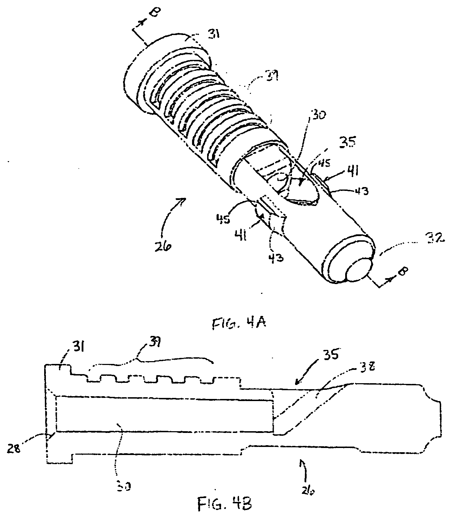

Referring now to the drawings in detail, wherein like numerals indicate the same elements throughout the views, FIGS. 1-3 illustrate a first embodiment for a suturing locking device 20 of the present description which is intended to be coupled to endoscope for insertion within a body. Suture locking device 20 deploys a knotting element during an endoscopic surgical procedure to effectively lock one or more pieces of suture material and prevent the material from dislodging within the patient's body. As shown in the Figures, suture locking device 20 comprises a substantially cylindrical, longitudinal launching member 24. The distal portion of launching member 24 comprises an inner locking member 26, which is shown in greater detail in FIGS. 4A and 4B. A flange 31 is preferably located at the distal tip of inner locking member 26. Flange 31 includes an opening into an axially-extending bore or channel 30. Bore 30 is sized to allow a suture material 36 to be inserted therethrough. The opening to bore 30 may be tapered, as indicated by reference numeral 28, to facilitate the threading of suture material 36 into the bore. Launching member 24 also includes an opening 35 from the proximal end of bore 30 to the exterior of the launching member, to enable suture material 36 to exit the member. Opening 35 may be angled, as indicated by reference numeral 38, to guide suture material 36 in a proximal exit direction along the outer surface of device 20.

The outer circumference of inner locking member 26 comprises an uneven surface area that engages suture material 36 when the knotting element is deployed. In the embodiment shown, the uneven surface area comprises a plurality of spaced indentations, as indicated by reference numeral 39. Indentations 39 are spaced apart distal of suture opening 35 to engage the proximal portion of suture material 36 as the material is looped back distally during firing. In addition to spaced indentations, other types of sculpted surface areas may also be utilized on the outer circumference of inner locking member 26 for engaging suture material 36 during firing. A pair of positional stops 41 are located on opposite sides of inner locking member 26 adjacent to suture opening 35. Positional stops 41 are shaped with a ramped proximal side 43 and a squared off distal end 45. The ramped proximal side 43 enables an outer locking member, which will be described below, to pass distally over stops 41 during firing to engage inner locking member 26. Once the outer locking member passes over positional stops 41 and onto the distal end of inner locking member 26, the squared off distal end 45 of the stops blocks the outer locking member from moving proximally and disengaging from the knotting element.

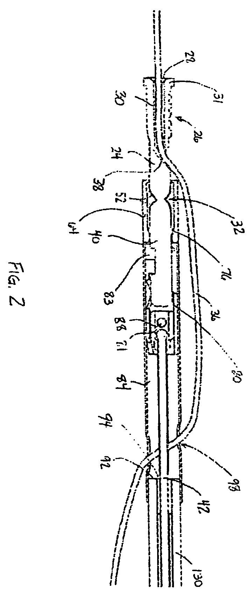

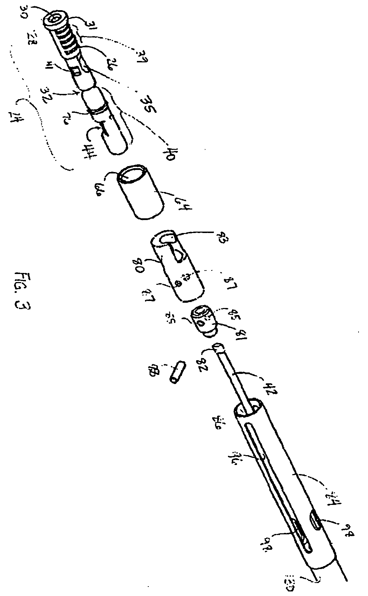

As shown in FIGS. 2 and 3, the proximal end of launching member 24 comprises an anchor section or knot tying element 40. Anchor section 40 comprises a longitudinally-extending, cylindrical shaft with a rounded proximal end. Anchor section 40 connects launching member 24 to a drive cable 42 so that the member may be moved by the cable during firing. A semi-circular cutout or recess 44 is formed in the outer surface of anchor section 40. Cutout 44 is shaped to engage a mating lip on a drive cable connector, which will be described below, when launching member 24 is inserted into device 20.

Between the inner locking member 26 and anchor section 40 of launching member 24, is a fracture section 32 shown in FIGS. 2 and 3. Fracture section 32 comprises an area of reduced diameter along the longitudinal length of launching member 24. In fracture section 32, the diameter of launching member 24 is substantially less than the diameter of the remaining length of the member, so as to form a weak point in the member. When pressure is applied to launching member 24 during firing, the reduced diameter in fracture section 32 causes structural failure at this point, thereby separating the distal end of launching member 24, including inner locking member 26, from anchor section 40 of the launching member. The particular diameter utilized within fracture section 32 will vary depending upon the diameter of the distal and proximal portions of launching member 24, as well as the strength of the particular materials used in manufacturing the launching member. The longitudinal length of fracture section 32 is preferably minimized to the shortest length necessary to ensure fracturing. The distal and proximal portions of launching member 24 adjacent to fracture section 32 have a parabolic shape, as indicated by reference numeral 52 in FIG. 2, to concentrate the stress on launching member 24 within the fracture section. In the embodiment described above, launching member 24 is formed as a single unit from a biocompatible plastic material such as, for example, polyetheretherketone (PEEK). In addition to PEEK, other biocompatible materials such as, for example, Vectra, may be used to form the launching member.

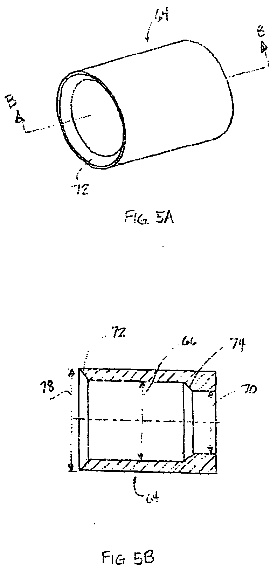

A cylindrical outer locking member 64 is disposed about the outer periphery of launching member 24. As shown in greater detail in FIGS. 5A and 5B, the inner surface of outer locking member 64 is separated into a first inner diameter 66 and a second, smaller inner diameter 70. The distal opening to first inner diameter 66 is tapered, as shown at 72, to provide a lead-in guide for inner locking member 26. The proximal end of first inner diameter 66 is also angled inwardly towards second inner diameter 70 to form an end stop 74. A detent 76 is formed on the outer circumference of launching member 24, as shown in FIGS. 2 and 3, for retaining outer locking member 64 in an unlocked position while suture locking device 20 is advanced to the suture site. In an unfired position, end stop 74 rests in contact with detent 76 on launching member 24 to position outer locking member 64 proximal of inner locking member 26 and suture opening 35. The outer diameter of inner locking member 26, including indentations 39, is greater than second inner diameter 70 of outer locking member 64. This diameter differential between inner and outer locking members 26, 64 forms a positional stop, causing inner locking member 26 to contact end stop 74 as launching member 24 moves proximally during firing. The contact between inner locking member 26 and end stop 74 terminates proximal movement of the inner locking member, and prevents the inner locking member from traveling completely through outer locking member 64.

First inner diameter 66 is selected to provide a clearance between the inner surface of outer locking member 64 and the outer surface of inner locking member 26 that is sufficient to deform suture material 36 between the opposing surfaces when the inner and outer locking members are joined into a knotting element. Additionally, indentations 39 along the outer surface of inner locking member 26 increase the contact area between suture material 36 and the inner locking member. Second inner diameter 70 extends proximally from end stop 74 to the proximal end of outer locking member 64. In an unlocked position, second inner diameter 70 surrounds launching member 24 proximal of detent 76. The reduced size of second inner diameter 70 prevents outer locking member 64 from moving distally along launching member 24 and prematurely locking prior to firing. The outer diameter 78 of outer locking member 64 is sized to allow the member and suture material to concurrently pass through a 2.8mm working channel of an endoscope.

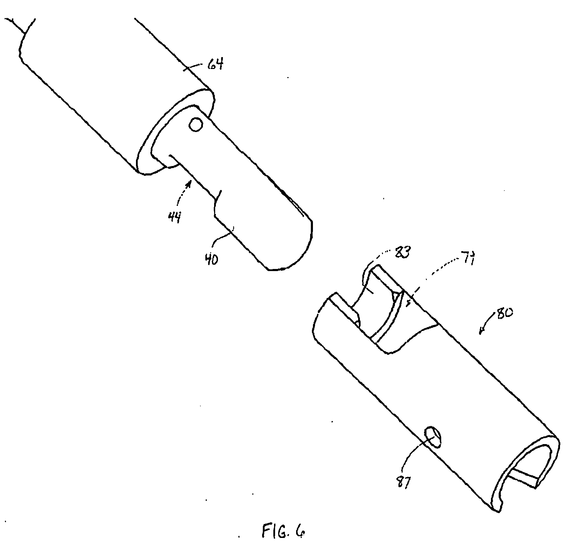

As shown in FIG. 6, anchor section 40 of launching member 24 is inserted into a cylindrically-shaped adaptor 80. The distal end of adaptor 80 is open or at least partially cut away, as indicated by reference numeral 79, to accommodate anchor section 40 when launching member 24 is loaded into the adaptor. A semi-circular lip 83 protrudes into the inner diameter of adaptor 80. Lip 83 engages cutout 44 of launching member 24 to operatively connect the launching member and adaptor 80. As shown in FIGS. 2 and 3, a cable connector 81 is disposed in the proximal end of adaptor 80 for connecting drive cable 42 to the adaptor and, correspondingly, to launching member 24. Drive cable 42 includes a coined distal end 82 for attachment within cable connector 81. The larger size of coined end 82 locks the cable within connector 81. A pin 88 is inserted through adaptor 80 and connector 81 via pairs of openings 85, 87. Openings 85, 87 extend through the connector and adaptor respectively in a direction perpendicular to the axial length of device 20. Pin 88 retains cable connector 81 within adaptor 80.

A cylindrical housing 84 extends proximally along the device axis from outer locking member 64. Housing 84 includes an open, distal end 86 that surrounds the outer circumference of adaptor 80. The inner diameter of open distal end 86 is selected to enable adaptor 80 to move freely in an axial direction within housing 84. The axial length of housing 84 is sufficient to allow adaptor 80 to pull a substantial length of launching member 24 into the housing during firing, thereby assuring that the tension applied to drive cable 42 is fully transferred to fracture section 32. The distal end of housing 84 also serves as a proximal end stop 90 for outer locking member 64. End stop 90 maintains outer locking member 64 in a fixed position during firing, thereby enabling inner locking member 26 to travel proximally into the first inner diameter 66 of the outer locking member. As inner locking member 26 travels into first inner diameter 66, outer locking member 64 plastically deforms around the inner locking member due to the limited clearance between the outer surface of the inner locking member and the inner surface of the outer locking member.

In an exemplary embodiment, the clearance between the opposing surfaces of the inner and outer locking members 26, 64 is approximately 0.1 mm for a monofilament suture material having a diameter of 0.2 mm. This clearance within the locked knotting element is sufficient to deform the suture material between the opposing locked surfaces, as well as deform outer locking member 64 about the exterior of inner locking member 26. The clearance between the inner and outer locking members may vary, however, depending upon the type of suture material being joined. Preferably, the clearance between the opposing locked surfaces is less than the diameter of the suture material, thereby assuring deformation of the material and increased friction between the suture material and mating surfaces of the knotting element.

As shown in FIGS. 1 and 3, housing 84 includes a pair of longitudinal side slots 96 through the outer diameter of the housing. The ends of pin 88 extend beyond adaptor openings 87 into side slots 96. Each side slot 96 aligns with one of the exposed ends of pin 88 so that the pin ends ride along the slots during firing. The movement of pin 88 through slots 96 properly orients adaptor 80 to prevent drive cable 42 from rotating within housing 84 as the cable moves linearly during firing. Spaced around housing 84 from side slots 96 are a pair of holes 98 that provide a passageway for suture material 36 to pass through housing 84 for in-line tensioning and cutting, as will be described in more detail below.

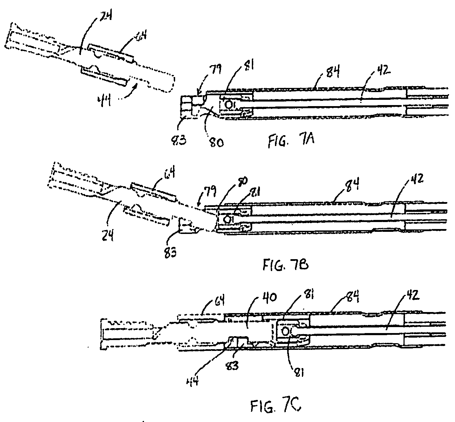

To load launching member 24 into device 20, adaptor 80 and cable connector 81 are first advanced distally until the partially open end of adaptor 80 extends beyond the open distal end of housing 84, as shown in FIG. 7A. Launching member 24 with outer locking member 64 loaded thereon (referred to herein as the knot tying element), is then inserted into the distal end of adaptor 80, by angling anchor section 40 of the launching member into adaptor opening 79, as shown in FIG. 7B. As anchor section 40 is angled into adaptor 80, cutout 44 of the anchor section moves into position above lip 83 of the adaptor. Once cutout 44 is in position over lip 83, launching member 24 is lowered down into adaptor 80 until the launching member is axially aligned with adaptor 80 and housing 84, as shown in FIG. 7C. Once launching member 24 is lowered, lip 83 engages cutout 44 to enable adaptor 80 to pull the launching member proximally when tension is applied to the adaptor through drive cable 42.

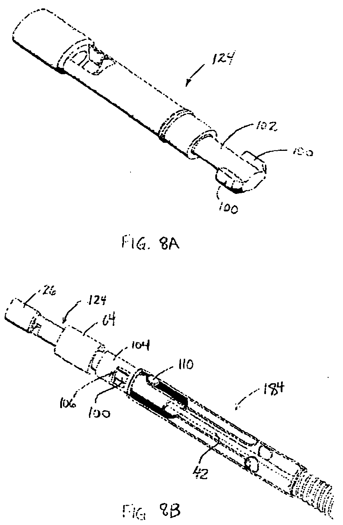

FIGS. 8A and 8B illustrate an alternative embodiment for attaching drive cable 42 to a launching member. In this embodiment, the launching member and adaptor are both modified to form a reloadable connection therebetween. As shown in FIG. 8A, a modified launching member 124 includes a plurality of bayonets 100. Bayonets 100 extend from a post 102 at the proximal end of launching member 124. Drive cable 42 is in turn securely attached, such as by welding or coining, to a ferrule 104, having a plurality of circumferential openings 106. Ferrule 104 is attached by a pin 110 to the open distal end of a modified housing 184. To attach drive cable 42 to launching member 124, ferrule 104 is threaded over the proximal end of the launching member such that openings 106 pass between bayonets 100. To lock ferrule 104 to launching member 124, the launching member is rotated relative to the ferrule until bayonets 100 reach openings 106. As bayonets 100 reach openings 106, the bayonets snap through the openings, locking launching member 124 to ferrule 104, as shown in FIG. 8B. As drive cable 42 is pulled proximally during firing, the interaction between bayonets 100 and openings 106 pulls launching member 124 and, thus, inner locking member 26, proximally into outer locking member 64. To release launching member 124 from ferrule 104, the launching member and modified housing 184 (with the attached ferrule) are twisted in opposite directions, causing bayonets 100 to disengage from openings 106. Once bayonets 100 are disengaged, ferrule 104 with attached drive cable 42 may be removed from launching member 124 and reattached to a new launching member by inserting ferrule 104 over the proximal end of the new launching member and twisting the bayonets into openings 106.

Returning now to FIG. 2, a catheter 130 such as, for example, a Bowden cable, is attached to the proximal end of adaptor 84. The inner diameter of the open proximal end of adaptor 84 may be sized to permit a light interference fit with catheter 130, to enable the adaptor and remaining aspects of suture locking device 20 to be detached from the catheter. Alternatively, suture locking device 20 may be permanently joined to catheter 130 at the open proximal end of adaptor 84.

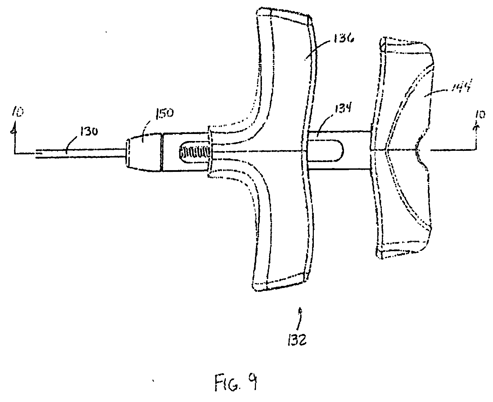

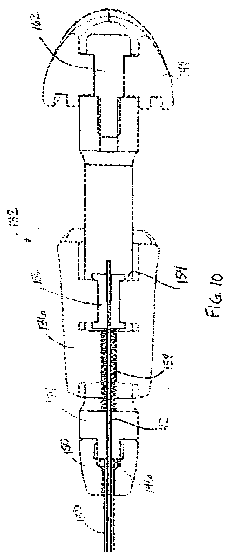

FIG. 9 illustrates the proximal end of catheter 130 and an exemplary handle 132 for deploying a knotting element from suture locking device 20. Handle 132 is attached at the proximal end of drive cable 42 for applying tension to the cable. Handle 132 comprises a longitudinal body portion 134, as well as a grip portion 136 for engaging the surgeon's fingers during operation of device 20. A thumb guide 144 is located at the proximal end of handle 132. As shown in greater detail in FIG. 10, the distal end of handle 132 includes an outer clamp 150 having a center bore for passage of catheter 130. A ring 146 is welded to the proximal end of catheter 130 and retained between clamp 150 and handle body 134 to secure the coil. Drive cable 42 extends proximally beyond clamp 150 and catheter 130 into a center bore 154 of handle body 134. A retaining member 156 is longitudinally disposed in bore 154 of handle body 134. Grip 136 is attached to retaining member 156 to move the retaining member within handle body 134 in response to pressure applied to the grip by the surgeon.

Drive cable 42 extends into a center bore within retaining member 156. The proximal end of drive cable 42 is secured within retaining member 156 by an attachment mechanism, such as, for example, a piece of metal tubing crimped to the end of the cable. Drive cable 42 is locked within retaining member 156 so as to move with the retaining member along the longitudinal axis of handle body 134. A resilient member 158 extends about drive cable 42 between the proximal end of handle body 134 and retaining member 156. Resilient member 158 serves to bias cable connector 81 into a proximal position within adaptor 80. An attachment mechanism 162 is lodged in the proximal end of handle body 134 to attach thumb guide 144 to the handle body, and to allow for rotation of the guide relative to the handle body. Tension is applied to drive cable 42 by pulling proximally on grip 136. As grip 136 moves proximally, retaining member 156 moves proximally within bore 154 of handle body 134, due to the connection between the grip and retaining member. As retaining member 156 moves proximally, the length of drive cable 42 is pulled proximally, increasing the tension on the cable. The increased tension on drive cable 42 is transferred to launching member 24 via the interconnection between cable connector 81, adaptor 80, and launching member 24. Handle body bore 154 is sized to allow drive cable 42 to be pulled a sufficient distance to pull inner locking member 26 into outer locking member 64, as well as separate the knotting element from launching member 24.

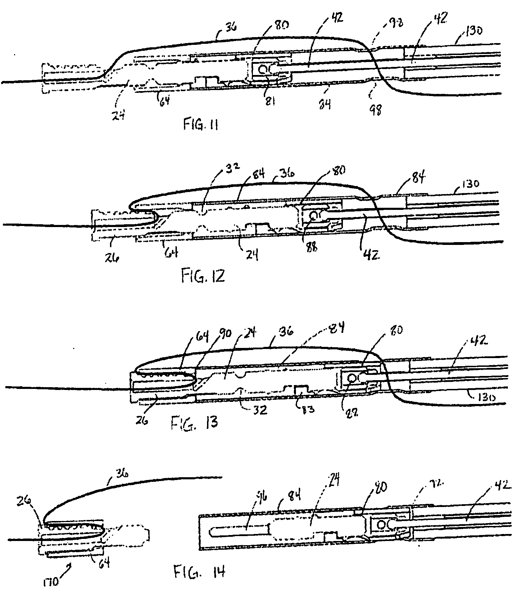

To deploy a knotting element from suture locking device 20, the device is introduced into the working channel of an externalized endoscope in an initial, unfired position. Suture locking device 20 is advanced through the working channel of the endoscope until inner and outer locking members 26, 64 are visible beyond the distal end of the scope. Suture material 36 that has been externalized out the patient's mouth (or other orifice or incision) is threaded into the distal end of inner locking member 26. The suture material is passed through bore 30 of inner locking member 26 and out through opening 35 of launching member 24. Upon exiting launching member 24, the ends of suture material 36 are passed through holes 98 in housing 84 and retrieved by the surgeon. The threaded path of suture material 36 is shown in FIG. 11.

Following threading of suture material 36 into device 20, the surgeon reintroduces the endoscope into the patient, and advances the scope to the suture site using the suture strands as a guide. In-line tension is maintained on suture material 36 while device 20 is passed towards the suture site by holding the externalized ends of the suture material. Once suture locking device 20 is in position at the suture site, tension is applied to suture material 36, as well as to handle 132, to fire the device. As grip 136 is drawn proximally, drive cable 42 is pulled proximally through handle 132, catheter 130 and housing 84. The movement of drive cable 42 applies tension to adaptor 80, which in turn pulls launching member 24 proximally due to the interaction between cut-out 44 and lip 83. As launching member 24 moves proximally along the device axis, inner locking member 26 is drawn into first inner diameter 66 of outer locking member 64, as shown in FIG. 12. This interaction between inner and outer locking members 26, 64 loops suture material 36 back in a distal direction, and holds the suture material tight, preventing the loss of tension.

As pressure continues to be applied to drive cable 42 by handle 132, inner locking member 26 is pulled further within outer locking member 64, causing the outer locking member to plastically deform about the inner locking member due to the small tolerance between the opposing surfaces of the locking members. As inner locking member 26 is pulled within outer locking member 64, suture material 36, which extends from the proximal end of inner member bore 30, is trapped between the outer diameter of the inner locking member and the first inner diameter 66 of the outer locking member, as shown in FIG. 13. Due to the limited clearance between the inner and outer locking member surfaces, suture material 36 is deformed therebetween. The uneven outer surface of inner locking member 26 increases the friction between suture material 36 and locking members 26, 64. Additionally, as suture material 36 is tensioned between inner and outer locking members 26, 64, flange 31 creates a right angle bend in the material, further increasing the strength of the suture lock within the knotting element.

As outer locking member 64 plastically deforms about inner locking member 26, launching member 24 and adaptor 80 move proximally within housing 84, with the outer ends of pin 88 moving through side slots 96. As adaptor 80 moves into the proximal end of housing 84, the proximal edge of the adaptor contacts the portion of suture material 36 extending through the housing between holes 98. The contact between the edge of the advancing adaptor 80 and suture material 36 severs the suture material within housing 84. FIG. 14 shows the distal separated end of suture material 36 after the proximal edge of adaptor 80 has moved proximally beyond holes 98 and completely severed the suture material.

After severing, the distal end of suture material 36 is locked between inner and outer locking members 26, 64, while the proximal portion of the suture material extends from a hole 98 of housing 84. As tension continues to be applied to drive cable 42 by way of handle 132, inner locking member 26 is prevented from further proximal movement within outer locking member 64 by end stop 74. Likewise, the locked inner and outer locking members 26, 64 become seated against end stop 90 of housing 84, and thereby prevented from further proximal movement. Once inner and outer locking members 26, 64 have reached the respective proximal stop positions, the further application of tension to launching member 24 via drive cable 42 generates a material failure or break at fracture section 32 of the launching member. The breaking tension of fracture section 32 is greater than the force required to plastically deform outer locking member 64 over inner locking member 26. The difference in tension force assures that inner and outer locking members 26, 64 are joined in the knotting element prior to detachment of the inner locking member from the remaining portion of launching member 24. As launching member 24 breaks apart at fracture section 32, the locked inner and outer members 26, 64 are detached from launching member 24 to form a separate knotting element 170, shown in FIG 14. In the knotting element 170, flange 31 and positional stops 41 prevent outer locking member 64 from moving relative to inner locking member 26. Accordingly, inner and outer locking members 26, 64 remain fixed in position within the body with suture material 36 deformed therebetween.

After separating from inner locking member 26, the remaining portion of launching member 24 is propelled proximally until pin 88 contacts the proximal end of side slots 96, thereby stopping further proximal movement of the launching member. After firing, adaptor 80 and the remaining portion of launching member 24, shown in FIG. 14, are removed from the body through the endoscope, leaving knotting element 170 at the suture site. After the remaining portions of device 20 have been removed from the body, the device may be reloaded with a new launching member, as described above, and the firing process repeated, to lock additional pieces of suture material.

The invention is defined in the appended claims that follow :

## Claims

1. A suture locking device (20) comprising:
a. an adapter (80, 84) for insertion into a body, said adapter (80, 84) having a proximal end, a distal end (79) and a lip (83) within said distal end (79) projecting towards an interior of said distal end (79), wherein said distal end (79) of said adaptor (80, 84) is at least partially cut away;
b. a knot tying element (24) releasably attachable to said distal end (79) of said adapter (80, 84), said knot tying element (24) having a distal and a proximal end (40), said proximal end (40) of said knot tying element (24) having a recess (44) thereon, said proximal end (40) of said knot tying element (24) being insertable by angling said proximal end (40) of said knot tying element (24) within said at least partially cut away distal end (79) of said adapter (80, 84) and then repositioning said knot tying element (24) into said adaptor (80, 84) such that said knot tying element (24) is axially aligned with said adaptor (80, 84) and said lip (83) and said recess (44) mate with each other to form a releasable lock.

2. The suture locking device (20) of claim 1 wherein said knot tying element (24) further comprises a locking element (26) having a bore (30) adapted for a suture (36) to run therethrough, said locking element (26) having an outer surface with a plurality of indentations (39) disposed thereon.

3. The suture locking device (20) of claim 1 wherein said distal end of said adapter (80, 84) has a first hole (98) on a first side of said adapter and a second hole (98) on a second side of said adapter wherein a suture (36) can go through said two holes (98) and wherein said holes (98) have centers which are at different locations along a longitudinal axis of said adapter (80, 84).

4. The suture locking device (20) of claim 1 further comprising a catheter (130) having a distal end attached to said proximal end of said adapter (80, 84).

5. The suture locking device (20) of claim 4 further comprising a drive cable (42) extending along and within said catheter (13), said drive cable (42) having a distal end attached to said proximal end of said adapter (80, 84).

6. The suture locking device (20) of claim 1 wherein said adapter (80, 84) is coupled to an endoscope.

7. The suture locking device (20) of claim 6 wherein said adapter (80, 84) is inserted into the working channel of an endoscope.

8. The suture locking device (20) of claim 1 wherein said adapter (80, 84) is substantially cylindrical.

9. The suture locking device (20) of claim 8 wherein said adapter (80, 84) has an outer diameter less than 2.8mm.

10. The suture locking device (20) of claim 1 wherein said knot tying element (24) further comprises a locking element (26) having a bore (30) with a suture running therethrough, said locking element (26) having an outer surface with a plurality of indentations (29) disposed thereon and wherein said locking element (26) comprises polyetheretherketone.

## Patentansprüche

1. Nahtmaterialarretierungsvorrichtung (20), umfassend:
a. einen Adapter (80, 84) zur Einführung in einen Körper, wobei der Adapter (80, 84) ein proximales Ende, ein distales Ende (79) und eine Lippe (83) in dem distalen Ende (79) hat, die zu einem Inneren des distalen Endes (79) hin vorragt, wobei das distale Ende (79) des Adapters (80, 84) mindestens teilweise weggeschnitten ist,
b. ein Knotenknüpfelement (24), das freigebbar an dem distalen Ende (79) des Adapters (80, 84) anbringbar ist, wobei das Knotenknüpfelement (24) ein distales und ein proximales Ende (40) hat, wobei das proximale Ende (40) des Knotenknüpfelements (24) eine Aussparung (44) daran hat, wobei das proximale Ende (40) des Knotenknüpfelements (24) eingeführt werden kann, indem das proximale Ende (40) des Knotenknüpfelements (24) winkelförmig in das mindestens teilweise weggeschnittene distale Ende (79) des Adapters (80, 84) eingeführt und dann das Knotenknüpfelement (24) in den Adapter (80, 84) neu positioniert wird, so dass das Knotenknüpfelement (24) axial auf den Adapter (80, 84) ausgerichtet ist und die Lippe (83) und die Aussparung (44) zur Bildung einer freigebbaren Arretierung ineinander eingreifen.

2. Nahtmaterialarretierungsvorrichtung (20) nach Anspruch 1, wobei das Knotenknüpfelement (24) ferner ein Arretierelement (26) mit einer Bohrung (30) umfasst, durch die ein Faden (36) laufen kann, wobei das Arretierelement (26) eine Außenfläche mit einer Vielzahl von daran angeordneten Vertiefungen (39) hat.

3. Nahtmaterialarretierungsvorrichtung (20) nach Anspruch 1, wobei das distale Ende des Adapters (80, 84) ein erstes Loch (98) an einer ersten Seite des Adapters und ein zweites Loch (98) an einer zweiten Seite des Adapters hat, wobei ein Faden (36) durch die beiden Löcher (98) gehen kann und wobei die Löcher (98) Mitten haben, die an verschiedenen Stellen entlang einer Längsachse des Adapters (80, 84) liegen.

4. Nahtmaterialarretierungsvorrichtung (20) nach Anspruch 1, ferner umfassend einen Katheter (130) mit einem distalen Ende, das am proximalen Ende des Adapters (80, 84) angebracht ist.

5. Nahtmaterialarretierungsvorrichtung (20) nach Anspruch 4, ferner umfassend ein Antriebskabel (42), das sich entlang und in dem Katheter (13) erstreckt, wobei das Antriebskabel (42) ein am proximalen Ende des Adapters (80, 84) angebrachtes distales Ende hat.

6. Nahtmaterialarretierungsvorrichtung (20) nach Anspruch 1, wobei der Adapter (80, 84) an ein Endoskop gekoppelt ist.

7. Nahtmaterialarretierungsvorrichtung (20) nach Anspruch 6, wobei der Adapter (80, 84) in den Arbeitskanal eines Endoskops eingeführt ist.

8. Nahtmaterialarretierungsvorrichtung (20) nach Anspruch 1, wobei der Adapter (80, 84) im Wesentlichen zylindrisch ist.

9. Nahtmaterialarretierungsvorrichtung (20) nach Anspruch 8, wobei der Adapter (80, 84) einen Außendurchmesser von weniger als 2,8 mm hat.

10. Nahtmaterialarretierungsvorrichtung (20) nach Anspruch 1, wobei das Knotenknüpfelement (24) ferner ein Arretierelement (26) mit einer Bohrung (30) umfasst, durch die ein Faden läuft, wobei das Arretierelement (26) eine Außenfläche mit einer Vielzahl von daran angeordneten Vertiefungen (29) hat und wobei das Arretierelement (26) Polyetheretherketon umfasst.

## Revendications

1. Dispositif (20) de blocage de sutures, comprenant :
a. un adaptateur (80, 84) pour insertion dans un organisme, ledit adaptateur (80, 84) ayant une extrémité proximale, une extrémité distale (79) et une lèvre (83) à l'intérieur de ladite extrémité distale (79), qui saille vers l'intérieur de ladite partie distale (79), dans laquelle ladite partie distale (79) dudit adaptateur (80, 84) est au moins partiellement découpée ;
b. un élément (24) pour faire des noeuds, pouvant être fixé amovible à ladite extrémité distale (79) dudit adaptateur (80, 84), ledit élément (24) pour faire des noeuds ayant une extrémité distale et une extrémité proximale (40), ladite extrémité proximale (40) dudit élément (24) pour faire des noeuds comportant sur elle un évidement (44), ladite extrémité proximale (40) dudit élément (24) pour faire des noeuds pouvant être insérée par une opération consistant à incliner ladite extrémité proximale (40) dudit élément (24) pour faire des noeuds à l'intérieur de ladite extrémité distale (79) au moins partiellement découpée dudit adaptateur (80, 84) et ensuite remettre en place ledit élément (24) pour faire des noeuds dans ledit adaptateur (80, 84) de telle sorte que ledit élément (24) pour faire des noeuds est aligné axialement avec ledit adaptateur (80, 84) et ladite lèvre (83) et ledit évidement (44) s'accouplent l'un avec l'autre pour constituer un blocage amovible.

2. Dispositif (20) de blocage de sutures selon la revendication 1, dans lequel ledit élément (24) pour faire des noeuds comprend en outre un élément de blocage (26) comportant un alésage (30) conçu pour qu'une suture (36) le traverse, ledit élément de blocage (26) comportant une surface extérieure avec une pluralité d'indentations (39) disposées sur elle.

3. Dispositif (20) de blocage de sutures selon la revendication 1, dans lequel ladite extrémité distale dudit adaptateur (80, 84) comporte un premier trou (98) sur un premier côté dudit adaptateur et un second trou (98) sur le second côté dudit adaptateur, dans lequel une suture (36) peut passer par lesdits deux trous (98) et dans lequel lesdits trous (98) ont des centres qui se trouvent à des endroits différents le long d'un axe longitudinal dudit adaptateur (80, 84).

4. Dispositif (20) de blocage de sutures selon la revendication 1, comprenant en outre un cathéter (130) ayant une extrémité distale fixée à ladite extrémité proximale dudit adaptateur (80, 84).

5. Dispositif (20) de blocage de sutures selon la revendication 4, comprenant en outre un câble de commande (42) s'étendant le long et à l'intérieur dudit cathéter (13), ledit câble de commande (42) ayant une extrémité distale fixée à ladite extrémité proximale dudit adaptateur (80, 84).

6. Dispositif (20) de blocage de sutures selon la revendication 1, dans lequel ledit adaptateur (80, 84) est accouplé à un endoscope.

7. Dispositif (20) de blocage de sutures selon la revendication 6, dans lequel ledit adaptateur (80, 84) est inséré dans le canal opérateur d'un endoscope.

8. Dispositif (20) de blocage de sutures selon la revendication 1, dans lequel ledit adaptateur (80, 84) est sensiblement cylindrique.

9. Dispositif (20) de blocage de sutures selon la revendication 8, dans lequel ledit adaptateur (80, 84) a un diamètre extérieur inférieur à 2,8 mm.

10. Dispositif (20) de blocage de sutures selon la revendication 1, dans lequel ledit élément (24) pour faire des noeuds comprend en outre un élément de blocage (26) comportant un alésage (30) avec une suture le traversant, ledit élément de blocage (26) comportant une surface extérieure avec une pluralité d'indentations (29) disposées sur elle et dans lequel ledit élément de blocage (26) comprend de la polyétheréthercétone.
